# EUROPÄISCHE PATENTSCHRIFT

(11) **EP 1 358 139 B1**
(45) Veröffentlichungstag und Bekanntmachung des Hinweises auf die Patenterteilung: **11.04.2007**
(21) Anmeldenummer: 02719721.9
(22) Anmeldetag: 24.01.2002
(51) Int. Cl.: C07C 5/22, C07C 11/21

(54) **THERMISCHE ISOMERISIERUNG VON LYCOPIN**
THERMAL ISOMERIZATION OF LYCOPENE
ISOMERISATION THERMIQUE DU LYCOPENE

(30) Priorität: 26.01.2001 DE 10103708
(43) Veröffentlichungstag der Anmeldung: 05.11.2003
(73) Patentinhaber: BASF Aktiengesellschaft, 67056 Ludwigshafen (DE)
(72) Erfinder: WEGNER, Christoph, 67281 Kirchheim (DE); JOHN, Michael, 67245 Lambsheim (DE)
(86) Internationale Anmeldenummer: PCT/EP2002/000708
(87) Internationale Veröffentlichungsnummer: WO 2002/072509

(56) Entgegenhaltungen:
- EP-A- 0 382 067
- EP-A- 0 895 997

## Beschreibung

Die vorliegende Erfindung betrifft ein Verfahren zur thermischen Isomerisierung einer Mischung von all-E-Lycopin und seiner Z-Isomere in beliebiger Zusammensetzung. Lycopin ist ein Carotinoid, das natürlich u.a. in der Tomate vorkommt.

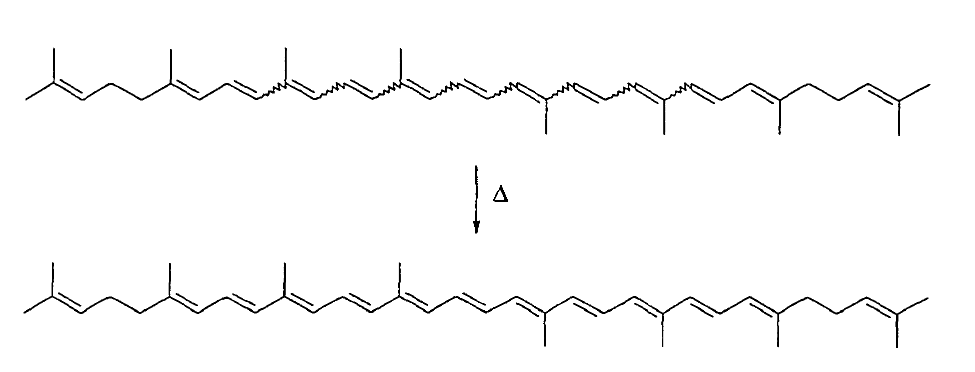

Es gibt bis heute keine Methode, mit der man ein Gemisch von Z-Isomeren oder auch einzelne Z-Isomere von Lycopin effizient in die all-E-Form überführen kann. Versuche dieses photochemisch oder thermisch zu tun, führen stets zu einem Gemisch verschiedener Lycopin-Isomere.

Die verstärkte Bildung von Z-Isomeren bei den bisherigen Versuchen, Lycopin photochemisch oder thermisch in die all-E-Form zu überführen, ist darauf zurückzuführen, dass das Lycopinmolekül stark S-förmig verdrillt ist. Somit gewinnt das Lycopin als all-E-Isomer nur einen geringen thermodynamischen Energievorteil, der bei Carotinoiden, die planare Konformation besitzen, signifikant höher liegt als bei den entsprechenden Z-Isomeren. Diese lassen sich dann oft thermisch in die all-E-Form isomerisieren (z.B. Astaxanthin). Lycopin, welches diesen Energievorteil der all-E-Form nicht besitzt, bildet daher ein Isomerengleichgewicht, in dem all-E-Lycopin im Vergleich zu den vielen möglichen Z-Isomeren eine energetisch nahezu gleichberechtigte Isomerenform ist, mit der Folge stark sinkender all-E-Werte bei Isomerisiserungsversuchen (abfallend bis Erreichen des Gleichgewichts).

Bei der Synthese von Lycopin entstehen bis zu 50 % Z-Isomere, was darauf zurückzuführen ist, dass das eingesetzte C₁₅-Phosphonium, welches zweimal an C₁₀-Dialdehyd angeknüpft wird, ein E/Z-Verhältnis von 4 : 1 besitzt und der ebenfalls eingesetzte C₁₀-Dialdehyd ein E/Z-Verhältnis von 96: 4 bis 97: 3 aufweist.

Im weiteren versteht man unter E/Z-Lycopin, all-E-Lycopin mit einem beliebigen Anteil an Z-Isomeren.

Aus diesem Grund setzen alle beschriebenen Synthesen von all-E-Lycopin (z.B. EP 895 997, EP 382 067) darauf, schon während der Synthese die Bildung von Z-Isomeren zu verhindern. Möglich ist dies nur durch aufwendige Synthesen, in die teilweise auch E-isomerenreine, teure Bausteine eingebracht werden müssen.

In der EP 382 067 ist ein Verfahren beschrieben, nach dem C15-Phosphoniumsalze niederer Alkansäuren als Zwischenprodukte hergestellt werden, da die Salze starker Säuren grundsätzlich schlechte E/Z-Selektivitäten und geringe Ausbeuten von Lycopin ergeben. Die Salze der Alkansäuren müssen vor der abschließenden Wittig-Olefinierung in einem aufwendigen Verfahren mittels Anionenaustausch wieder in die Chloride überführt werden. Zur Erzielung eines hohen E/Z-Verhältnisses im Lycopin ist zusätzlich eine Abtrennung von (Z)-Anteilen des Phosphoniumsalzes durch Kristallisation erforderlich.

Aufgabe der Erfindung war es eine effiziente Isomerisierungsmethode der Z-Form in die all-E-Form zu entwickeln, die die im Stand der Technik beschriebenen Nachteile nicht aufweist sowie den Einsatz der kostengünstigeren E/Z-Synthesebausteine ermöglicht.

Erfindungsgemäß gelöst wurde die Aufgabe durch ein Verfahren zur thermischen Isomerisierung einer Mischung von all-E-Lycopin und seiner Z-Isomere in beliebiger Zusammensetzung unter Steigerung des all-E-Anteils, das dadurch gekennzeichnet ist, dass die Isomerisierung in einem polaren Lösungsmittel stattfindet, in dem Lycopin nur schwer löslich ist.

Gegenstand der Erfindung ist somit ein Verfahren zur thermischen Isomerisierung von all-E-Lycopin und seinen Z-Isomeren in beliebiger Zusammensetzung unter Steigerung des all-E-Anteils, dadurch gekennzeichnet, dass die Isomerisierung in einem polaren Lösungsmittel stattfindet.

Die bisherigen Versuche, Lycopin zu isomerisieren, wurden stets in Lösungen durchgeführt. Da die thermodynamische Stabilität des all-E-Isomeren des Lycopins durch eine sehr verdrillte Konformation aber hier keinen oder nur einen verschwindend geringen energetischen Vorteil hat, stellte sich stets ein Gemisch von vielen Z-Isomeren ein.

In dem erfindungsgemäßen Verfahren wird eine Suspension von Lycopin in einem polaren Lösungsmittel, in dem Lycopin nur schwer löslich ist eingesetzt.

Als polare Lösungsmittel werden Alkohole, wie C₁-C₈-Alkohole, Diole, Polyole, Amide, Carbonate, Sulfoxide oder Wasser eingesetzt.

C₁-C₈-Alkohole sind beispielsweise Methanol, Ethanol, Ethylenglykol, Glycerin, Propanol, Isopropanol, Butanol, tert.-Butanol, Pentanol, Hexanol, Heptanol oder Octanol, vorzugsweise werden Methanol, Ethanol oder Butanol eingesetzt. Als Diol kann beispielsweise Ethylenglykol eingesetzt werden. Unter Polyolen versteht man beispielsweise Polyethylenglykol. Amide sind beispielsweise Formamid, Acetamid, Methylformamid, Methylacetamid, Dimethylformamid, Dimethylacetamid oder γ-Butyrolacton. Unter Carbonaten versteht man beispielsweise Ethylencarbonat oder Propylencarbonat. Als Sulfoxid kann beispielsweise Dimethylsulfoxid verwendet werden.

Der Effekt, dass all-E-Lycopin sehr viel besser kristallisiert als die Z-Isomere wird in dem erfindungsgemäßen Verfahren in einem Lösungsmittel, in dem sich Lycopin fast nicht löst ausgenutzt. Hierzu muß das Lösungsmittel so polar sein, dass sich die Z-Isomere kaum in Lösung aufhalten können, sondern durch Oberflächenaffinität sich als amorphe bzw. ölige Schicht um die all-E-Kristalle legen. In diesem Fall ist bei ausreichend hoher Temperatur eine selektive Isomerisierung der Z-Isomere in dieser Schicht möglich, da die im Kristall gebundenen all-E-Isomere eine wesentlich höhere Isomerisierungsaktivierungsenergie besitzen. Durch die lokale Nähe zu den all-E-Kristallen wird ein im stetig dynamischen Isomerisierungsprozess immer wieder zufällig entstehendes all-E-Isomer sofort in den Kristall integriert und damit einer Rückisomerisierung in eine Z-Form entzogen. In Summe kann man das Isomerisierungsgleichgewicht so zum all-E-Isomer hin verschieben.

Vorzugsweise sollte die Löslichkeit von Lycopin bei Raumtemperatur unter 0,1 % liegen. Die Isomerisierungstemperatur liegt zwischen 40 und 180°C, vorzugsweise zwischen 60 und 120°C. Die Isomerisierung kann sowohl unter Normaldruck als auch unter Druck, vorzugsweise bei Drücken von 1 bis 6 bar durchgeführt werden.

Zur Herstellung einer Suspension von Lycopin in einem polaren Lösungsmittel, in dem sich Lycopin nur schwer löst, wird direkt nach der Wittig-Reaktion per destillativem Lösungsmittelaustausch auf dieses Lösungsmittel umgestellt oder die Wittig-Reaktion zum Lycopin wird direkt in diesem polaren Lösungsmittel durchgeführt.

Nun kann in verschiedenen Mengenverhältnissen Lycopin zum polaren Lösungsmittel, vorzugsweise als 5 bis 20 %ige Suspension von Lycopin im polaren Lösungsmittel unter Erhitzen isomerisiert werden.

Nun können sich drei verschiedene Varianten anschließen: Zum einen kann die Suspension direkt unter Erhitzen isomerisiert werden. Alternativ wird das Volumen an abdestilliertem Dichlormethan durch Zugabe von Alkohol, beispielsweise Methanol ersetzt und anschließend durch Erhitzen isomerisiert. Bei der dritten Variante wird ein Lösungsmitteltausch auf beispielsweise n-Butanol vorgenommen und dann isomerisiert.

Ausgewertet werden können die Versuche, indem man die Kristalle nach Abkühlen der Suspension abfiltriert, nachwäscht, trocknet, auswiegt und per UV-Messung den Gehalt bestimmt.

Erzeugt man beispielsweise durch Lösungsmitteltausch von z.B. Dichlormethan, in dem Lycopin synthetisiert wird, auf Methanol eine solche Suspension, in der eine beliebige Mischung von all-E-Lycopin mit Z-Isomeren vorliegen kann, und erhitzt diese unter Rückfluss oder auch unter Überdruck auf höhere Temperaturen, so unterliegen nur die nicht-kristallinen Lycopinisomere einer permanenten Isomerisierung, nicht hingegen das Lycopin, welches im Kristall (all-E) eingebaut ist. Immer wenn sich das frei vorliegende Z-Isomer in ein all-E-Isomer umwandelt, wird es in den Kristall eingebaut und ist somit keiner Rückisomerisierung mehr zugänglich.

Eine Mischung von 50 % all-E-Lycopin mit 50 % Z-Isomeren konnte so bis auf einen all-E-Gehalt von 75 % gesteigert werden. Eine vollständige Isomerisierung lässt sich nicht erreichen, da das 5Z-Isomere ähnlich gut kristallisiert wie all-E-Lycopin, so dass das Verhältnis von all-E zu 5Z nicht beeinflusst werden kann. Alle anderen Isomere wandeln sich in diese beiden um. Die Kristallausbeute nach Abfiltration an Lycopin konnte so um 27 bis 35 % gesteigert werden.

Da Lycopin in den meisten Lösungsmitteln nur schwer löslich ist, kann dieses Verfahren prinzipiell in fast jedem Lösungsmittel durchgeführt werden. Die einzige Bedingung ist, dass bei der gewählten Isomerisierungstemperatur mehr als 70 %, besser mehr als 90 %, des all-E-Lycopins in kristalliner Form vorliegen müssen.

Durch das Entstehen größerer, definierter Kristalle während der Isomerisierung nahm die Reinheit der Kristalle erheblich zu. In der Folge konnten schon nach der ersten Kristallisation Phosphorwerte von <100 ppm erzielt werden. Die ist höchst erstaunlich, da aus einer Lösung kristallisiert wurde, in der sich zwei Äquivalente Triphenylphosphan befanden. Normale Lycopin-Qualitäten (ohne Isomerisierung) besitzen um die 1000 ppm Phosphor.

Insgesamt lassen sich damit die folgenden Vorteile durch die erfindungsgemäße thermische Isomerisierungsmethode für Lycopin erzielen:
- deutlich höhere Kristallausbeute an kristallinem Lycopin
- bei der Synthese von Lycopin können kostengünstigere Einsatzstoffe eingesetzt werden, da diese nicht mehr E-isomerenrein sein müssen
- Steigerung des all-E-Anteils
- Steigerung des Gehaltes

Die folgenden Beispiele sollen die Erfindung näher erläutern, ohne sie jedoch darauf einzuschränken

### Beispiele

### Beispiel 1

Zu einer Lösung von Lycopin (44,0 g) mit einem all-E-Gehalt von 53 % in Dichlormethan (317 g) wurde bei 40°C Methanol (330 ml) gegeben und das Dichlormethan anschließend abdestilliert (als Azeotrop mit MeOH, 94:6), wobei das abdestillierende Dichlormethanvolumen kontinuierlich durch in den Reaktor einlaufendes Methanol ersetzt wurde. Die entstandene Suspension von Lycopin in Methanol wurde nun 40 h unter Rückfluss erhitzt, wobei nach 16 h und nach 40 h eine HPLC-Analyse durchgeführt wurde: Nach 16 h lag der all-E-Anteil schon bei 67 % und nach 40 h bei 68 %. Der 5Z-Anteil lag die ganze Zeit konstant bei 18 %. Die Suspension wurde nun auf 0°C abgekühlt, abfiltriert und mit Methanol gewaschen (4 x 100 ml). Nach Trocknen im Stickstoffgas-Strom konnten 42,3 g Lycopin-Kristallisat mit einem Gehalt von 86 % und einem all-E-Anteil von 76,3 % isoliert werden. Dies entspricht 36,4 g Lycopin.

### Beispiel 2

Zu einer Lösung von Lycopin (44,0 g) mit einem all-E-Gehalt von 53 % in Dichlormethan (317 g) wurde bei 40°C Methanol (330 ml) gegeben und das Dichlormethan anschließend abdestilliert (als Azeotrop mit MeOH, 94:6). Die entstandene Suspension von Lycopin in Methanol wurde nun 40 h unter Rückfluss erhitzt, wobei nach 16, 23 und 40 h eine HPLC-Analyse durchgeführt wurde: Nach 16 h lag der all-E-Anteil bei 64 %, nach 23 h bei 66 % und nach 40 h bei 71 %. Die Suspension wurde nun auf 0°C abgekühlt, abfiltriert und mit Methanol gewaschen (4 x 100 ml). Nach Trocknen im Stickstoffgas-Strom konnten 42,3 g Lycopin-Kristallisat mit einem Gehalt von 92 % und einem all-E-Anteil von 73,4 % isoliert werden. Dies entspricht 38,9 g Lycopin.

### Beispiel 3

Zu einer Lösung von Lycopin (44,0 g) mit einem all-E-Gehalt von 53 % in Dichlormethan (317 g) wurde bei 40°C Methanol (330 ml) gegeben und das Dichlormethan anschließend abdestilliert (als Azeotrop mit MeOH, 94:6), wobei das abdestillierende Dichlormethanvolumen kontinuierlich durch in den Reaktor einlaufendes Methanol ersetzt wurde. Die entstandene Suspension von Lycopin in Methanol wurde nun 12 h unter Eigendruck (ca. 3 bar) auf 95°C erhitzt. Die Suspension wurde danach auf 0°C abgekühlt, abfiltriert und mit Methanol gewaschen (4 x 100 ml). Nach Trocknen im Stickstoffgas-Strom konnten 39,4 g Lycopin-Kristallisat mit einem Gehalt von 97 % und einem all-E-Anteil von 81,2 % isoliert werden. Dies entspricht 38,2 g Lycopin.

### Beispiel 4

Zu einer Lösung von Lycopin (44,0 g) mit einem all-E-Gehalt von 53 % in Dichlormethan (317 g) wurde bei 40°C Methanol (330 ml) gegeben und das Dichlormethan anschließend abdestilliert (als Azeotrop mit MeOH, 94:6). Die entstandene Suspension von Lycopin in Methanol wurde nun 12 h unter Eigendruck (ca. 3 bar) auf 95°C erhitzt. Die Suspension wurde danach auf 0°C abgekühlt, abfiltriert und mit Methanol gewaschen (4 x 100 ml). Nach Trocknen im Stickstoffgas-Strom konnten 44,0 g Lycopin-Kristallisat mit einem Gehalt von 99 % und einem all-E-Anteil von 79,6 % isoliert werden. Dies entspricht 43,6 g Lycopin.

### Beispiel 5

Zu einer Lösung von Lycopin (44,0 g) mit einem all-E-Gehalt von 53 % in Dichlormethan (317 g) wurde bei 40°C Methanol (330 ml) gegeben und das Dichlormethan anschließend abdestilliert (als Azeotrop mit MeOH, 94:6), wobei das abdestillierende Dichlormethanvolumen kontinuierlich durch in den Reaktor einlaufendes Methanol ersetzt wurde. Die entstandene Suspension von Lycopin in Methanol wurde nun 6 h unter Eigendruck (ca. 5 bar) auf 120°C erhitzt. Die Suspension wurde danach auf 0°C abgekühlt, abfiltriert und mit Methanol gewaschen (4 x 100 ml). Nach Trocknen im Stickstoffgas-Strom konnten 40,3 g Lycopin-Kristallisat mit einem Gehalt von 85 % und einem all-E-Anteil von 87,5 % isoliert werden. Dies entspricht 35,3 g Lycopin.

### Beispiel 6

Zu einer Lösung von Lycopin (44,0 g) mit einem all-E-Gehalt von 53 % in Dichlormethan (317 g) wurde bei 40°C 1-Butanol (330 ml) gegeben und das Dichlormethan anschließend abdestilliert, wobei das abdestillierende Dichlormethanvolumen kontinuierlich durch in den Reaktor einlaufendes 1-Butanol ersetzt wurde. Die entstandene Suspension von Lycopin in 1-Butanol wurde nun 15 h auf 95°C erhitzt. Die Suspension wurde danach auf 0°C abgekühlt, abfiltriert und mit Methanol gewaschen (4 x 100 ml). Nach Trocknen im Stickstoffgas-Strom konnten 32,9 g Lycopin-Kristallisat mit einem Gehalt von 100 % und einem all-E-Anteil von 87,8 % isoliert werden. Dies entspricht 32,9 g Lycopin.

### Beispiel 7

Vergleich: Ohne den Isomerisierungsschritt konnte lediglich eine Auswaage an kristallinem Lycopin von 30,0 g mit einem Gehalt von 95 % und einem all-E-Anteil von 75,6 % erzielt werden. Dies entspricht 28,5 g Lycopin.

## Patentansprüche

1. Verfahren zur thermischen Isomerisierung einer Mischung von all-E-Lycopin und seiner Z-Isomere in beliebiger Zusammensetzung unter Steigerung des all-E-Anteils, **dadurch gekennzeichnet, dass** die Isomerisierung in einem polaren Lösungsmittel stattfindet.

2. Verfahren gemäß Anspruch 1, **dadurch gekennzeichnet, dass** die Isomerisierung zwischen 60 und 180°C stattfindet.

3. Verfahren gemäß Anspruch 1 oder 2, **dadurch gekennzeichnet, dass** die Löslichkeit von all-E-Lycopin bei Raumtemperatur in dem polaren Lösungsmittel kleiner 0,1 % liegt.

4. Verfahren gemäß einem der Ansprüche 1 bis 3, **dadurch gekennzeichnet, dass** als Lösungsmittel C₁-C₈-Alkohole, Diole, Polyole, Amide, Carbonate, Sulfoxide oder Wasser verwendet werden.

5. Verfahren gemäß einem der Ansprüche 1 bis 4, **dadurch gekennzeichnet, dass** als Lösungsmittel Methanol, Ethanol, Isopropanol oder Butanol verwendet werden.

6. Verfahren gemäß einem der Ansprüche 1 bis 5, **dadurch gekennzeichnet, dass** bei der gewählten Isomerisierungstemperatur mehr als 70 % des all-E-Lycopins in kristalliner Form vorliegt.

## Claims

1. A process for the thermal isomerization of a mixture of all-E-lycopene and its Z isomers of any composition to increase the proportion of all-E, wherein the isomerization takes place in a polar solvent.

2. The process according to claim 1, wherein the isomerization takes place at between 60 and 180°C.

3. The process according to claim 1 or 2, wherein the solubility of all-E-lycopene in the polar solvent at room temperature is less than 0.1%.

4. The process according to any of claims 1 to 3, wherein C₁-C₈-alcohols, diols, polyols, amides, carbonates, sulfoxides or water are used as solvent.

5. The process according to any of claims 1 to 4, wherein methanol, ethanol, isopropanol or butanol are used as solvent.

6. The process according to any of claims 1 to 5, wherein more than 70% of the all-E-lycopene is in crystalline form at the chosen isomerization temperature.

## Revendications

1. Procédé d'isomérisation thermique d'un mélange de lycopène tout E et de ses isomères Z en composition quelconque, avec augmentation de la fraction tout E, **caractérisé en ce que** l'isomérisation a lieu dans un solvant polaire.

2. Procédé suivant la revendication 1, **caractérisé en ce que** l'isomérisation a lieu entre 60 et 180°C.

3. Procédé suivant la revendication 1 ou 2, **caractérisé en ce que** la solubilité à la température ambiante de lycopène tout E dans le solvant polaire est inférieure à 0,1 %.

4. Procédé suivant l'une des revendications 1 à 3, **caractérisé en ce que**, comme solvant, on utilise des alcools en C₁-C₈, des diols, des polyols, des amides, des carbonates, des sulfoxydes ou de l'eau.

5. Procédé suivant l'une des revendications 1 à 4, **caractérisé en ce que**, comme solvant, on utilise du méthanol, de l'éthanol, de l'isopropanol ou du butanol.

6. Procédé suivant l'une des revendications 1 à 5, **caractérisé en ce que**, à la température d'isomérisation choisie, plus de 70 % du lycopène tout E se présentent sous forme cristalline.
